# DEMANDE DE BREVET EUROPEEN

(11) **EP 1 060 731 A1**
(43) Date de publication de la demande: **20.12.2000**
(21) Numéro de dépôt: 99630053.9
(22) Date de dépôt: 18.06.1999
(51) Int. Cl.: A61K 6/033, A61L 27/12

(54) **Matériau de comblement bio-actif, procédé d'utilisation de ce matériau et appareil pour la mise en oeuvre du procédé**

(71) Demandeur: Bone & Joint Research S.A., 3644 Kayl (LU)
(72) Inventeur: Moulin, Jean, 3644 Kayl (LU); Munting, Evrard, 1390 Biez (BE)
(74) Mandataire: Schmitz, Jean-Marie

(57) **Abrégé**

Pâte de comblement (1) bio-active comprenant une poudre (6) de liant hydraulique phosphocalcique et des granulés (7) d'hydroxyapatite ou de fluoroapatite à structure cristalline de préférence hexagonale stoéchiométrique de taille entre 10 et 2000 microns mélangés dans une faible quantité de liquide réactif (8). L'invention concerne également un procédé d'introduction de la pâte de comblement bio-active pour le remplissage de cavités ou d'espaces pour assurer le renfort, la réparation par fusion de tissus dentaires ou osseux ou la stabilisation d'un implant ainsi qu'une installation pour la mise en oeuvre du procédé.

## Description

La présente invention concerne un matériau de comblement bio-actif, un procédé d'utilisation de ce matériau et des appareils pour la mise en oeuvre du procédé.

La pâte de comblement bio-active selon l'invention comprend un liant hydraulique phosphocalcique, des grains d'hydroxyapatite de taille choisie entre 10 et 2000 µm (microns) selon les propriétés recherchées, et un liquide réactif biocompatible de préférence aqueux, dont la viscosité peut être réduite et rendue fluide par un procédé et appareil vibratoire, afin de permettre le remplissage de cavités ou d'espaces, en vue de leur comblement pour assurer le renfort, la réparation par fusion des tissus dentaires ou osseux, par exemple, ou encore la stabilisation d'un implant, après durcissement de ladite pâte in situ.

Comme domaines d'applications et d'utilisations de la pâte de comblement selon l'invention on peut citer, sans être limité à ceux-ci: les implants chirurgicaux, dentaires, vétérinaires; ostéoporose, tumeurs, fractures, arthroplasties, révisions, réparation, renfort, comblement, des tissus osseux; arthrodèse d'espaces articulaires dégénératifs, scellements, ancrages, ostéo-induction, ostéo-conduction, greffes, fusion.

Les substances permettant le comblement de cavités osseuses existent sous deux familles génériques selon leur mode de mise en oeuvre:
- Des ciments polymères ou des résines, à effet de durcissement.
- Des granulés ou des blocs à tailler, à effet d'intégration cellulaire.

1/ Les ciments utilisés en implantologie sont dérivés des composites dentaires obtenus par mélange d'une poudre de résine de synthèse et d'un durcisseur plus ou moins liquide. Outre leur réaction exothermique qui nécrose les tissus périphériques pendant la phase de durcissement, la toxicité des solvants permettant la prise est une cause importante et bien documentée de risque per-opératoire.

Les résines thermodurcissable, ou polymérisant sous l'action des ultra violets sont impossibles à mettre en oeuvre en milieu chirurgical stérile.

De plus ces ciments de synthèse de par leur composition chimique relativement inerte, s'ils sont tolérés par l'os et les tissus vivants, ne peuvent prétendre participer à leur régénération ou leur réparation.

2/ Les granulés utilisés pour combler les cavités osseuses sont de deux types:

- soit inertes, comme les céramiques d'alumine, donc associés à de l'os de greffe de préférence autogène.

- soit bio-actifs car leur composition est proche de la phase minérale de l'os et sont reconnus par les cellules ostéogènes. Sous forme de granulés, ils s'incorporent lentement par colonisation et résorption, au détriment de leur résistance mécanique. Sous forme de blocs, ils offrent une meilleure tenue mécanique dans le temps, mais au détriment d'une intégration médiocre principalement périphérique.

Dans tous les cas ces matériaux bio-actifs, blocs ou granulés, ne peuvent assurer une stabilité immédiate et nécessitent une immobilisation complémentaire, le temps de leur assimilation par l'organisme.

Le EP-A-0 422 719 décrit un biomatériau de comblement osseux ou dentaire à base d'apatite, à base de phosphate de calcium oxygéné ayant une structure apatique qui possède des espèces oxygénées à des degrés d'oxydation supérieurs ou égaux à - 2.

Le EP-A-0 401 793 décrit un matériau de remplacement de tissus durs vivants comprenant un matière céramique contentant du CaO et SiO₂ comme constituants principaux et facultativement du MgO et ne contient pas de phospate de calcium.

Le FR-A-2 684 003 décrit un matériau implantable bio-dégradable constitué par un mélange d'hydroxylapatite ou d'apatite contenant d'autres phosphates de calcium ou de la chaux qui mélangés avec de l'acide lactique et de l'eau donne une pâté malléable qui durcit par la suite. Un mélange gélatine-apatite-glycérol-eau donne par traitement à basse pression une éponge osseuse de densité et dureté variables.

Le EP-A-0 564 369 décrit un matériau pour prothèse osseuse biorésorbable, contenant des particules de carbonate de calcium, provenant par exemple de squelette de corail, dispersées au sein d'une matrice polymère, lesdites particules ayant des dimensions inférieures à 1 mm et représentant de 40 à 70% du poids total, et ledit polymère étant un polymère biorésorbable.

Le EP-A-0 417 018 divulgue un matériau bioréactif du type verre ou verre partiellement cristallisé, pour prothèse osseuse ou implant dentaire, qui contient en % poids, 5 à 14 de Na₂O, 0 à 12% de P₂O₅, 49 à 57% de SiO₂, le solde étant constitué d'au plus 33% d'un mélange de CaO et CaF₂, ce dernier étant compris entre 0,5 et 7%.

Le FR-A-2 693 716 décrit un procédé d'obtention d'hydroxylapatite phosphocalcique (HAP). Ce procédé consiste à mélanger, sous forme de poudre, un phosphate monocalique (MCP), un phosphate tricalcique (TCP) et un phosphate tétracalcique (TTCP), et à faire réagir le mélange avec de l'eau en présence d'un glycérophosphate soluble dans l'eau, en particulier glycérophosphate de sodium (NaGP). Le procédé de l'invention permet d'obtenir une hydroxyapatite stoéchiométrique ou quasi stoéchiométrique, avec des temps de prise modulables compatibles avec une utilisation chirurgicale, en comblement en particulier.

Le propos de l'invention est de permettre la mise en place dans les cavités osseuses d'accès difficile et par une incision minimale ou si possible en percutané par un procédé arthroscopique, d'une pâte de comblement comprenant une poudre et des granulés mélangés dans une faible quantité de liquide réactif, dont la forte viscosité associée à la présence de grains rend l'injection impossible sans l'action d'ondes vibratoires dont la longueur ou fréquence sont choisies selon les proportions du mélange ; ladite pâte présente une composition physico-chimique permettant son durcissement en présence des fluides biologiques et reproduisant sensiblement la phase minérale de l'os afin de permettre son ostéo-intégration. Le pâte de comblement bio-active de la présente invention est décrite dans la revendication 1.

Le procédé d'application de la pâte de comblement bio-active de la présente invetion est décrit dans la revendication 7.

L'appareil pour la mise en oeuvre du procédé selon l'invention et décrit dans la revendication 8.

Pour que l'invention puisse être mieux comprise, référence est faite aux dessins, dnas lesquels:
- la figure 1 représente une installation pour le remplissage d'une cavité dans un os par la pâte de comblement selon la présente invention.
- la figure 2 représente un autre mode de réalisation pour le remplissage d'une cavité dans un os par la pâte de comblement selon la présente invention.
- la figure 3 réprésente encore un troisième mode de réalisation pour le remplissage d'une cavité dans un os par la pâte de comblement selon la présente invention.
- la figure 4 réprésente un quatrième mode de réalisation pour le remplissage d'une cavité dans un os par la pâte de comblement selon la présente invention.

La pâté de comblement (1) bio-active de la présente invention est obtenue par le mélange d'un matériau bio-actif de synthèse comprenant un liant hydraulique phosphocalcique sous forme de poudre (6) et des grains d'hydroxypatite (7), ou encore de fluoroapatite, à structure cristalline de préférence hexagonale stoéchiométrique donc faiblement résorbables, de taille chosie entre 10 et 2000 µm (microns) selon les propriétés recherchées, dans un liquide réactif initiateur de prise (8), de manière à former un ciment à prise en milieu aqueux dont les propriétés mécaniques en compression sont renforcées et maintenues pendant son assimilation par l'organisme, grâce à la présence desdits grains (7).

Les grains d'hydroxyapatite (7) ou de fluoroapatite, constituant les éléments durs, faiblement résorbables peuvent être composés de cristaux de préférence orientés de manière allongée sous forme par exemple d'aiguilles, pleines ou creuses, réalisant l'armature dudit ciment. Le liant hydraulique phosphocalcique sous forme de poudre (6) est réalisé de préférence par assemblage de petits cristaux de 1 à 100 nanomètres, ledit assemblage présentant une micro porosité comprise entre 10 et 20000 nanomètres, afin de faciliter la circulation du liquide réactif de prise (8) pendant la phase de durcissement, puis la circulation des fluides biologiques pendant la phase de résorption permettant d'obtenir leur saturation déclenchant la précipitation d'apatites non stoéchiométriques intégrant les protéines spécifiques de la matrice osseuse.

Des éléments ou matériaux peuvent être ajoutés afin d'organiser une macro-porosité comprise entre 50 et 600 micromètres dans la masse du ciment afin de faciliter les échanges ioniques, la vascularisation ou la capture de cellules souches ostéopoïétiques permettant par un effet ostéoconducteur la formation d'os.

Le liant hydraulique phosphocalcique sous forme de poudre (6) est constitué de préférence pour partie de phosphate bicalcique (BCP), de phosphate tricalcique (TCP), d'apatite carbonatée, d'hydroxyapatite (HA) non stoéchiométrique, de carbonate de calcium, de sulfate semi hydraté de calcium, ou d'au moins deux de ces éléments.

La viscosité de la pâte de comblement selon l'invention est réduite et est rendue plus fluide (2) par un procédé utilisant un appareil vibratoire (3) afin de permettre le renfort, la séparation par fusion des tissus dentaires ou osseux (5) ou encore la stabilisation d'un implant (9) après durcissement in situ de ladite pâte.

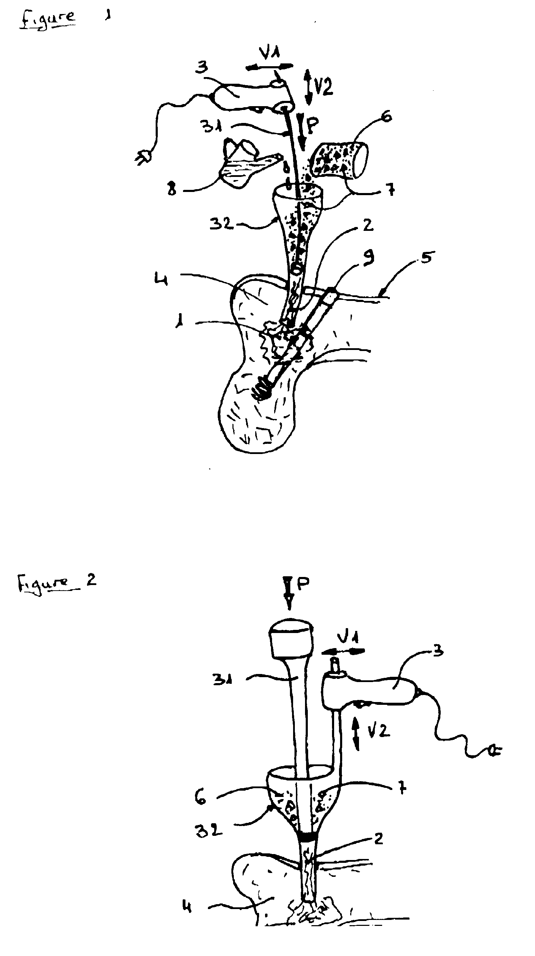

La figure 1 réprésente une installation pour mettre en oeuvre le procédé de remplissage, selon l'invention, d'une cavité (4) dans un os (5). Dans cette cavité peut se trouver par exemple un implant (9) à fixer en place. Un appareil vibratoire (3) crée une série d'ondes vibratoires (V1, V2) le long d'un poussoir (31) qui transmet les vibrations au mélange qui est formé en mettant en suspension la phase solide (6, 7) dans le liquide réactif de prise (8) dans un introducteur (32). Ceci rend la pâte (1) fluide (2) et sous l'influence de la poussée (P) qui est créée par l'ensemble, l'appareil vibratoire (3) et poussoir (31) la pâte fluide s'écoule facilement dns la cavité (4) et autour de l'implant (9) qu'elle contribue à stabiliser après durcissement in situ.

Ainsi qu'on l'a indiqué ci-dessus, la pâté de comblement (1) est constituée d'un mélange de liant hydraulique phosphocalcique sous forme de poudre (6) et de grains d'hydroxyapatite (7), ou encore de fluoroapatite, à structure cristalline auquel est ajouté un liquide réactif initiateur de prise (8).

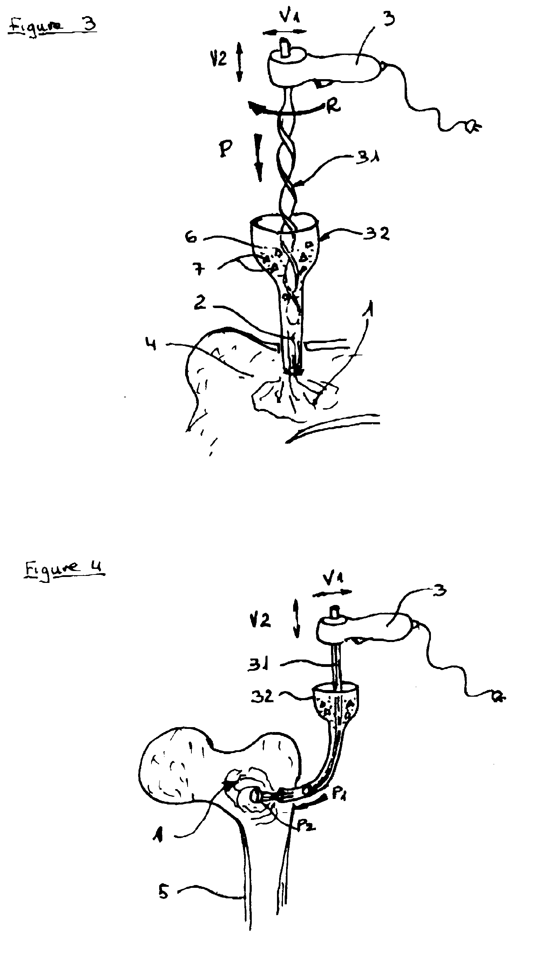

La figure 2 représente un autre mode de réalisation d'installation pour remplissage d'une cavité (4) dans un os (5) par la pâte de comblement (1) comprenant une poudre (6) et des granulés (7) mélangés dans un faible quantité de liquide réactif (8) dont la viscosité peut être réduite et rendue fluide (2) afin de permettre le remplissage de cavités (4) par un procédé vibratoire. Dans ce mode de réalisation l'appareil vibratoire (3, V1 et V2) est fixé directement sur l'introducteur (32) et le poussoir (31) exerce une pression (8) sur la pâte (1) à l'état fluide (2) pour l'injecter dans la cavité.

La figure 3 représents un mode de réalisation d'installation pour le remplissage d'une cavité d'un os (5), similaire à celui de la figure 1 à l'exception que le mouvement vibratoire (V1, V2) est transmit à un poussoir (31) en forme de vis d'Archimède dont la rotation (R) entraîne la pâte de comblement (1) dans l'introducteur (32) et ensuite vers la cavité (4). Dans ce cas l'appareil vibratoire incorpore un moteur permettant la rotation dudit poussoir en forme de vis d'Archimède.

Dans le mode de réalisation de la figure 4 on utilise un introducteur (32) courbe et un poussoir (31) flexible. Ce dernier transmet les vibrations (V1, V2) au mélange et son déplacement (P1 à P2) jusque dans l'os (5) facilite le remplissage de la cavité (4) même dans une position rétrograde.

L'invention a été décrite par des modes de réalisation préférés mais il est bien entendu que d'autres modifications peuvent être faites dans le cadre de l'invention qui n'est limitée que par les revendications.

## Revendications

1. Pâte de comblement (1) bio-active comprenant une poudre (6) et des granulés (7) mélangés dans une faible quantité de liquide réactif (8) dont la viscosité peut être réduite et rendue fluide (2) par un appareil et procédé vibratoire (V1 et V2), afin de permettre le remplissage de cavités (4) ou d'espace, pour assurer le renfort, la réparation par fusion des tissus dentaires ou osseux (5), ou encore la stabilisation d'un implant (9), après durcissement in situ de ladite pâte (1), celle-ci présentant une composition physico-chimique permettant son durcissement en présence des fluides biologiques et reproduisant sensiblement la phase minérale de l'os afin de permettre son ostéo-intégration.

2. Pâte de comblement (1) bio-active selon la revendication 1 obtenue par le mélange d'un matériau bio-actif de synthèse comprenant un liant hydraulique phosphocalcique sous forme de poudre (6) et des grains d'hydroxyapatite (7), ou encore de fluoroapatite, à structure cristalline de préférence hexagonale stoéchiométrique donc faiblement résorbables, de taille choisie entre 10 et 2000 microns selon les propriétés recherechées, dans un liquide réactif initiateur de prise (8) de préférence aqueux, de manière à former un ciment dont les propriétés mécaniques en compression sont renforcées, et maintenues pendant son assimilation par l'organisme, grâce à la présence desdits grains (7).

3. Pâte de comblement (1) bio-active selon les revendications 1 et 2 comprenant des grains d'hydroxyapatite (7), ou de fluoroapatite à structure cristalline de préférence hexagonale stoéchiométrique, de taille choisie entre 10 et 2000 microns, constituant les éléments durs, faiblement résorbables composés de cristaux de préférence orientés de manière allongée sous forme par exemple d'aiguilles, pleines ou creuses, réalisant l'armature dudit ciment, le choix du type d'aiguilles et/ou de leur diamètre permettant de réguler la vitesse de leur dissolution selon leur rapport masse/surface.

4. Pâte de comblement (1) bio-active selon les revendications 1 à 3 comprenant un liant hydraulique phosphocalcique sous forme de poudre (6) réalisé de préférence par assemblage de petits cristaux de 1 à 100 nanomètres, afin de faciliter la circulation du liquide réactif de prise pendant la phase de durcissement, puis la circulation des fluides biologiques pendant la phase de résorption permettant d'obtenir leur saturation déclenchant la précipitation d'apatites non stoéchimétriques intégrant les protéines spécifiques de la matrice osseuse.

5. Pâte de comblement (1) bio-active selon les revendications 1 à 4 à laquelle peuvent être ajoutés des éléments ou matériaux permettant d'organiser une macro-porosité comprise entre 50 et 600 micromètres dans la masse du ciment afin de faciliter les échanges ioniques, la vascularisation ou la capture de cellules souches ostéopoïétiques permettant par un effet ostéoconducteur la formation d'os.

6. Pâte de comblement (1) bio-active selon les revendications 1 à 5 comprenant un liant hydraulique phosphocalcique sous forme de poudre (6) constitué de préférence pour partie de phosphate bicalcique (BCP), de phosphate tricalcique (TCP), d'apatite carbonatée, d'hydroxyapatite (HA) non stoéchiométrique, de carbonate de calcium, de sulfate semihydraté de calcium, ou d'au moins deux de ces éléments.

7. Procédé d'introduction de la pâte de comblement bio-active des revendications 1 à 6 pour le remplissage de cavités (4) ou d'espaces pour assurer le renfort, la réparation par fusion des tissus dentaires ou osseux (5) ou la stabilisation d'un implant (9), après durcissement in situ de ladite pâte (1), ce procédé consistant à introduire la pâte par un effet vibratoire (V1 et V2) qui réduit la viscosité et rend la pâte plus fluide.

8. Installation pour la mise en oeuvre du procédé de la revendication 7 comprenant un appareil vibratoire (3) et éventuellement rotatoire créant des ondes vibratoires (V1, V2) transmettant les vibrations à un mélange formé en mettant en suspension une phase solide (6,7) dans le liquide réactif de prise (8) dans un introducteur (32) et comprenant un poussoir (31) pour faciliter l'introduction du mélange dans la cavité (4).

9. Installation selon la revendication 8, l'appareil vibratoire étant fixé au poussoir (31) créant une série d'ondes vibratoires qui transmet les vibrations au mélange formé en mettant en suspension la phase solide (6,7) dans le liquide réactif de prise (8) dans un introducteur (32) rendant aussi la pâte (1) fluide (2) et sous l'influence de la poussée (P) qui est créée par l'ensemble, appareil vibratoire (37) et poussoir (31) le pâte fluide s'écoule facilement dans la cavité (4).

10. Installation selon la revendication 8, l'appareil vibratoire (3) étant fixé directement à l'introducteur (32), le poussoir exerçant un pression (P) sur la pâte (1) pour l'injecter dans la cavité (4).

11. Installation selon la revendication 9, le poussoir (31) étant flexible pour pouvoir introduire la pâte dans un introducteur (32) ayant une forme courbée, le déplacement (P1 et P2) du poussoir jusque dans l'os facilitant le remplissage de la cavité (4) même dans une position rétrograde.

12. Installation selon la revendication 9, le poussoir étant en forme de vis d'Archimède dont la rotation (R) entraîne la pâte dans l'introducteur (32) vers la cavité (4). L'appareil vibratoire incorporant un moteur permettant la rotation dudit poussoir en forme de vis d'Archimède.
